(19) Europäisches Patentamt
European Patent Office
Office européen des brevets

(11) EP 0 816 326 B1

(12) EUROPÄISCHE PATENTSCHRIFT

(45) Veröffentlichungstag und Bekanntmachung des Hinweises auf die Patenterteilung:
**10.05.2000 Patentblatt 2000/19**

(51) Int Cl.$^7$: **C07C 227/18**, C08G 18/38, C09D 175/12

(21) Anmeldenummer: **97109989.0**

(22) Anmeldetag: **19.06.1997**

(54) **Verfahren zur Herstellung von Mono- und Polyasparaginsäureestern**

Process for preparing mono- and polyspartic acid esters

Procédé de préparation d'esters d'acide mono- ou polyaspartique

(84) Benannte Vertragsstaaten:
**AT BE CH DE ES FR GB IT LI NL**

(30) Priorität: **02.07.1996 DE 19626470**
**25.04.1997 DE 19717427**

(43) Veröffentlichungstag der Anmeldung:
**07.01.1998 Patentblatt 1998/02**

(73) Patentinhaber: **BAYER AG**
**51368 Leverkusen (DE)**

(72) Erfinder:
- **Kurek, Gerald, Dr.**
  **51373 Leverkusen (DE)**
- **Pedain, Josef, Dr.**
  **51061 Köln (DE)**
- **Halpaap, Reinhard, Dr.**
  **51519 Odenthal (DE)**
- **Sonntag, Michael, Dr.**
  **51519 Odenthal (DE)**

(56) Entgegenhaltungen:
**EP-A- 0 403 921** **EP-A- 0 596 360**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 816 326 B1

Printed by Jouve, 75001 PARIS (FR)

## Beschreibung

Die vorliegende Erfindung betrifft ein neues Verfahren zur Herstellung von Mono- und Polyasparaginsäureestern aus primären Mono- und Polyaminen und Maleinsäureestern unter Zuhilfenahme von Stickstoffatome enthaltenden Fünfring-Heteroaromaten als Katalysatoren sowie die Verwendung der auf die erfindungsgemäße Art und Weise erhaltenen Mono- und Polyasparaginsäureester als Reaktivkomponente für Polyisocyanate in Zweikomponenten-Polyurethan-Beschichtungsmitteln sowie bei der Herstellung von Polyurethan-Prepolymeren.

Zweikomponenten-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente, enthalten, sind seit langem bekannt. Sie eignen sich zur Herstellung von hochwertigen Überzügen, die hart, elastisch, abrieb- und lösemittelbeständig und vor allem auch witterungsstabil eingestellt werden können.

Innerhalb dieser 2K-PUR-Beschichtungstechnologie haben sich in letzter Zeit bestimmte, estergruppenhaltige, sekundäre Polyamine fest etabliert, die sich in Kombination mit Lackpolyisocyanaten insbesondere als Bindemittel in lösemittel armen oder -freien High Solid-Beschichtungsmitteln eignen und eine rasche Aushärtung der Beschichtungen bei niedrigen Temperaturen ermöglichen.

Bei diesen sekundären Polyaminen handelt es sich um die sogenannten Polyasparaginsäureester, wie sie in der EP-A-0 403 921 beschrieben werden. Ihre Verwendung allein oder im Gemisch mit weiteren, gegenüber Isocyanatgruppen reaktionsfähigen Komponenten in 2K-PUR-Beschichtungsmitteln wird in den EP-A 0 403 921, 0 639 628, 0 667 362, 0 689 881, US-A 5 214 086, EP-A 0 699 696, 0 596 360 und US-A 5 243 012 beschrieben.

Ein geeignetes Verfahren zur Darstellung dieser Polyasparaginsäureester stellt die Umsetzung entsprechender primärer Polyamine mit Malein- oder Fumarsäureestern der allgemeinen Formel $R^1OOC-CH=CH-COOR^2$ - wobei $R^1$ und $R^2$ für gleiche oder verschiedene organische Reste stehen - zu den sekundären Polyaminen dar. Aufgrund sterischer, struktureller und elektronischer Effekte weisen diese sekundären Aminogruppen gegenüber Isocyanatgruppen eine ausreichend verminderte Reaktivität auf, so daß eine sichere und handhabbare Abmischung von Polyisocyanaten und diesen Polyaminen und damit eine Verarbeitung als Bindemittel möglich ist.

Bei der bei dieser Herstellung der Polyasparaginsäureester ablaufenden Reaktion handelt es sich um eine Addition von primären Aminen an die aktivierte C-C-Doppelbindung vinyloger Carbonylverbindungen, die in der Literatur hinreichend beschrieben ist (vgl. Chem. Ber. 1946, *38*, 83; Houben Weyl, Meth. d. Org. Chemie Bd. 11/1, 272 (1957), Usp. Chimii 1969, *38*, 1933). Es zeigt sich jedoch, daß diese Umsetzung nach Ablauf der eigentlichen Syntheseoperation (z. B. 24 Stunden Rühren bei 60°C) je nach Art des primären Polyamins mehr oder weniger unvollständig ist. So beträgt der Umsetzungsgrad (gemessen am Gehalt des freien, nicht umgesetzten Malein- bzw. Fumarsäureesters, in den sich der Maleinsäureester durch Basenkatalyse umlagert) z. B. bei 1,6-Hexandiamin nach einem Tag ca. 90 - 93 %, während er bei cycloaliphatischen Polyaminen mit sterisch gehinderten primären Aminogruppen wie z.B. 4,4'-Diamino-3,3'-dimethyldicyclohexyl-methan (®Laromin C260) nur 77 % beträgt. Die (nahezu) vollständige Umsetzung wird erst mehrere Tage oder - wie beim ®Laromin C 260 - erst mehrere Monate nach der eigentlichen Syntheseoperation erreicht.

Vor Beendigung dieser "Reifezeit" besitzen diese Produkte erhöhte Anteile an noch nicht abreagierten primären Aminogruppen und sogar freien primären Polyaminen sowie in gleichem Maße an noch nicht abreagiertem Malein- oder Fumarsäureester. Dies führt aufgrund der heftigen Reaktion primärer Aminogruppen mit Isocyanatgruppen zur schlechten Handhabbarkeit während des Verarbeitungsprozesses und zu schlechten Schichteigenschaften wie z.B. mangelnder Kratzfestigkeit und schränkt damit den Einsatz dieser unreifen Produkte wesentlich ein. Nachteilig sind solche Produkte auch in toxikologischer Hinsicht.

Die Verlängerung der Reaktionszeit oder die Erhöhung der Reaktionstemperatur auf 80°C oder gar 100°C sind ohne weiteres nicht geeignet, die Reaktion zu beschleunigen, da sich hierbei die Farbzahl des Produktes drastisch erhöht.

In der EP-A 0 667 362 und US-A 5 243 012 wird eine Verlängerung der Topfzeit von 2K-PUR-Bindemitteln auf Basis von Polyisocyanaten und Polyasparaginsäureestern durch Zusatz von Zeolithen bzw. Organozinn(IV)-Verbindungen beschrieben. Diese Maßnahmen haben jedoch nur behelfsmäßigen Charakter und beeinträchtigen andere Eigenschaften des Bindemittels nachhaltig (Trübung, Beschleunigung der NCO/OH-Reaktion).

Es war daher die Aufgabe der Erfindung, durch Zusatz einer oder mehrerer, katalytisch wirksamer Verbindungen die Asparaginsäureestersynthese so zu beschleunigen, daß nach einer ökonomisch akzeptablen Synthesezeit Mono- und Polyasparaginsäureester erhalten werden, die den Anforderungen bezüglich Farbzahl, Handhabbarkeit und Reaktivität standhalten, ohne daß eine notwendige Lagerzeit des Produktes einzuhalten wäre.

Diese Aufgabe konnte überraschend durch Zusatz einer oder mehrerer katalytisch wirksamer Verbindungen gelöst werden. Zwar ist die Katalyse von Additionen an vinyloge Doppelbindungen bekannt (Organikum, S.502, 16. Auflage, VEB Deutscher Verlag der Wissenschaften, Berlin 1986), jedoch zeigte sich hierbei, daß die meisten der in Frage kommenden, katalytisch wirksamen Verbindungen nicht nur die Reaktion beschleunigen, sondern auch zu einer drastischen, nicht akzeptablen Farbvertiefung des Produktes führen.

Die Zusätze müssen demnach folgende Eigenschaften in sich vereinigen:

a) Sie müssen die Reaktion zur Herstellung der Polyasparaginsäureester deutlich beschleunigen,

b) sie dürfen keine Nebenreaktionen begünstigen, die unter Umständen auch noch zu einer Verfärbung des Produktes führen,

c) sie sollten sowohl eine Verfärbung des Reaktionsgemisches während als auch nach dem Syntheseprozeß, hier vor allem bei der Abmischung mit weiteren, gegenüber Isocyanatgruppen reaktiven Verbindungen, unterdrücken,

d) sie dürfen keinen Einfluß auf die Reaktion der Polyasparaginsäureester und gegebenenfalls weiterer Reaktivkomponenten mit den Polyisocyanaten ausüben, und

e) sie dürfen die Schichteigenschaften nicht negativ beeinflussen.

Jetzt wurde überraschend gefunden, daß Fünfring-Heteroaromaten mit zwei, drei oder vier Stickstoffatomen im Ringgerüst hervorragend geeignete Katalysatoren für die Darstellung von Mono- und Polyasparaginsäureestern aus primären Mono- und Polyaminen und Malein- bzw. Fumarsäureestern darstellen, die die oben genannten Bedingungen a) bis e) erfüllen.

Gegenstand der Erfindung ist ein Verfahren zur Herstellung von sekundären Mono- und Polyaminen der Formel (I)

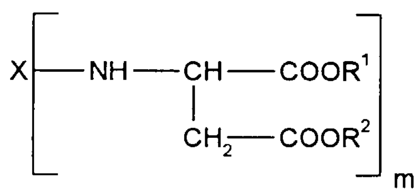 (I),

durch Umsetzung der entsprechenden Mono- oder Polyamine der allgemeinen Formel (II) (Komponente A)

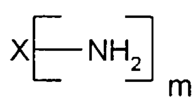 (II),

mit Verbindungen der allgemeinen Formel (III) (Komponente B)

$$R^1OOC\text{-}CH{=}CH\text{-}COOR^2 \qquad (III),$$

bei Temperaturen zwischen 0°C und 100°C, vorzugsweise 20 - 80°C und besonders bevorzugt 20 - 60°C, in Lösung oder lösemittelfrei, mit einem Verhältnis der Äquivalente der primären Aminogruppen der Komponente A zu den C=C-Doppelbindungsäquivalenten der Komponente B von 1:2 bis 1,5:1, wobei überschüssige Edukte im Anschluß an die Reaktion abdestilliert werden können,
dadurch gekennzeichnet, daß die Reaktion in Anwesenheit einer katalytisch und verfärbungsstabilisierend wirkenden Komponente C, bestehend aus einer oder mehreren Verbindungen der Formeln (IV) bis (XI)

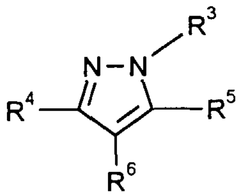

(IV),

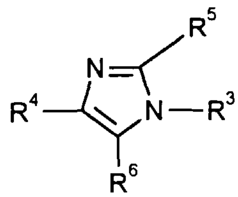

(V),

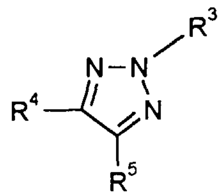

(VI),

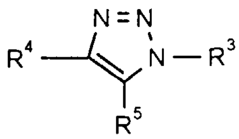

(VII),

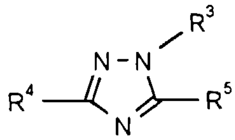

(VIII),

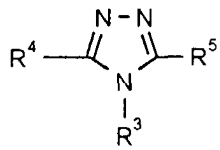

(IX),

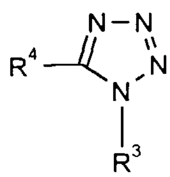

(X),

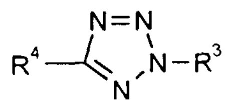

(XI),

abläuft,
wobei der Anteil der Komponente C am Reaktionsgemisch, bezogen auf den Festkörpergehalt des Reaktionsgemisches, 0,1 bis 10 Gew.-%, vorzugsweise 0,1 bis 5 Gew.-% und besonders bevorzugt 0,1 bis 2 Gew.-% beträgt, und wobei in den Formeln (I) bis (XI)

X für einen m-wertigen, gegebenenfalls ein oder mehrere Heteroatome (z.B. N, O, S) enthaltenden organischen Rest steht, wie er durch Entfernung der primären Aminogruppe bzw. -gruppen aus einem entsprechenden, (cyclo)aliphatisch gebundene Aminogruppen aufweisenden Mono- oder Polyamin des Molekulargewichtsbereichs 60 bis 6000, vorzugsweise 88 bis 322 erhalten werden kann, und der weitere gegenüber Isocyanatgruppen reaktive und/oder bei Temperaturen bis 100°C inerte funktionelle Gruppen enthalten kann,

$R^1$ und $R^2$ für gleiche oder verschiedene organische Reste, vorzugsweise für gleiche oder verschiedene Alkylreste mit 1 bis 18 Kohlenstoffatomen und ganz besonders bevorzugt für gleiche oder verschiedene Alkylreste mit 1 bis 8 Kohlenstoffatomen stehen,

$R^3$ für einen organischen, gegebenenfalls ein oder mehrere Heteroatome wie z.B. N, O, S aufweisenden organischen Rest mit 1 bis 24 C-Atomen, für die $NH_2$-Gruppe oder für ein Wasserstoffatom steht,

$R^4$, $R^5$ und $R^6$ für gleiche oder verschiedene, gegebenfalls ein oder mehrere Heteroatome wie N, O, S aufweisende organische Reste mit 1 bis 24 C-Atomen, die gegebenenfalls Bestandteil eines weiteren, gegebenenfalls gemeinsamen Ringsystemes sein können, für die $NH_2$-Gruppe und/oder für ein Wasserstoffatom stehen, und

m für eine ganze Zahl ≥ 1, bevorzugt ≥ 2 und besonders bevorzugt = 2 steht.

Gegenstand der Erfindung ist weiterhin ein Verfahren zur Herstellung von Überzügen durch Beschichtung beliebiger Substrate mit einem Beschichtungsmittel, welches als Bindemittel ein Zweikomponenten-System aus

a) einer Polyisocyanatkomponente und

b) einer gegenüber Polyisocyanaten reaktionsfähigen Reaktivkomponente,

sowie gegebenenfalls die aus der Beschichtungstechnologie bekannten Hilfs- und Zusatzmittel enthält, dadurch gekennzeichnet, daß die Bindemittelkomponente b) aus

b1) einer oder mehreren, erfindungsgemäß erhaltenen Verbindungen der allgemeinen Formel (I) oder aus Gemischen derartiger Verbindungen mit

b2) aus der Polyurethanchemie an sich bekannten, gegenüber Isocyanatgruppen reaktionsfähigen Reaktivkomponenten besteht,

wobei die Komponenten b1) und b2) in beliebigem Verhältnis zueinander stehen können, wobei das Äquivalentverhältnis von Isocyanatgruppen zu gegenüber Isocyanatgruppen reaktionsfähigen Gruppen bei 0,8:1 bis 20:1 liegt und die Aushärtung nach erfolgter Applikation auf dem Substrat bei Temperaturen von -20 bis + 80°C erfolgt, wobei in der Formel (I) X, $R^1$, $R^2$ und m die obengenannten Bedeutungen haben.

Gegenstand der Erfindung ist ebenfalls die Verwendung der erfindungsgemäß erhaltenen Mono- und Polyasparaginsäureester gegebenenfalls im Gemisch mit einer oder mehreren, weiteren gegenüber Isocyanatgruppen reaktionsfähigen Reaktivkomponenten als Reaktionspartner für Polyisocyanate bei der Herstellung von Harnstoff-, Urethan-, Allophanat- und/oder Biuretstrukturen aufweisenden Prepolymeren.

Für das erfindungsgemäße Verfahren zur Herstellung der sekundären Mono- und Polyamine der Formel (I) kommen als Komponente A der allgemeinen Formel (II) im Prinzip alle bekannten Mono- und Polyamine mit mindestens einer primären Aminogruppe in Betracht.

Als primäre Monoamine der allgemeinen Formel (II) (m=1) beispielsweise gut geeignet sind diejenigen Monoamine, die in ihrem organischen Rest X eine oder mehrere, weitere funktionelle Gruppen enthalten, die entweder gegenüber Isocyanatgruppen reaktiv oder inert sind. Während gegenüber Isocyanatgruppen reaktionsfähige funktionelle Gruppen wie Hydroxyl-, sek. Amino- und Carboxylreste zusätzliche Reaktionspartner für die Isocyanatgruppen darstellen, bieten die gegenüber Isocyanatgruppen inerten funktionellen Gruppen die Möglichkeit sowohl zu alternativen Vernetzungsmechanismen wie z. B. Feuchtigkeitshärtung als auch zu besserer Verträglichkeit mit weiteren Zusatzstoffen (Lösemitteln, Pigmente etc.) sowie zu besserer Haftung der Polyurethanbeschichtungen auf bestimmten Substraten. Beispielhaft, aber nicht ausschließlich, genannt seien Aminoalkylalkoxysilane und Aminoalkylalkylsilane des Typs $H_2N$- $R^7$-Si $R^8R^9R^{10}$ mit $R^8$, $R^9$ und $R^{10}$ als Chloratom, Alkyl- und/oder Alkoxyresten mit 1 bis 4 Kohlenstoffatomen und mit $R^7$ als Alkylenrest mit 2 bis 6 Kohlenstoffatomen sowie Aminoalkohole wie z. B. Ethanolamin.

Geeignete Polyamine der allgemeinen Formel (II) ($m \geq 2$) sind beispielsweise, aber nicht ausschließlich, Ethylendiamin, 1,2-Diaminopropan, 1,4-Diaminobutan, 2,5-Diamino-2,5-dimethylhexan, 1,5-Diamino-2-methylpentan (Dytec A), 1,6-Diaminohexan (HDA), 2,2,4- und/oder 2,4,4-Trimethyl-1,6-diaminohexan, 1,11-Diaminoundecan, 1,12-Diaminododecan, 1-Amino-3,3,5-trimethyl-5-aminomethylcyclohexan (IPDA), 2,4- und/oder 2,6-Hexahydrotoluylendiamin ($H_6$TDA), andere alkylsubstituierte Cyclohexandiamine, wie z. B. Isopropyl-2,4-und/oder 2,6-diaminocyclohexan, 2,4'- und/oder 4,4'-Diamino-dicyclohexylmethan (PACM 20), 3,3'-Dimethyl-4,4'-diaminodicyclohexylmethan (®Laromin C 260), die isomeren, eine Methylgruppe als Kernsubstituenten aufweisenden Diaminodicyclohexylmethane (= C-Monomethyl-diaminodicyclohexylmethane), 3(4)-Aminomethyl-1-methylcyclohexylamin (AMCA) oder auch, weniger bevorzugt, höhermolekulare Polyetherpolyamine mit aliphatisch gebundenen primären Aminogruppen, wie sie beispielsweise unter der Bezeichnung ®Jeffamin von der Firma Texaco vertrieben werden.

Bei der Komponente B der allgemeinen Formel (III) handelt es sich um die sogenannten Malein- und Fumarsäureester. Zur erfindungsgemäßen Herstellung der Mono- oder Polyasparaginsäureester der allgemeinen Formel (I) geeignete Malein- oder Fumarsäureester sind beispielsweise, aber nicht ausschließlich, Maleinsäuredimethylester, -diethylester, -di-n- oder -isopropylester, -di-n-butylester, -di-2-ethylhexylester oder die entsprechenden Fumarsäureester.

Bei der erfindungsgemäßen katalytisch wirksamen Komponente C handelt es sich im allgemeinen um eine oder mehrere Verbindungen aus der Reihe solcher Fünfring-Heteroaromaten, deren Ringgerüste zwei (Pyrazole der allgemeinen Formel (IV) und Imidazole der allgemeinen Formel (V)), drei (1,2,3-Triazole der allgemeinen Formeln (VI) und (VII) sowie 1,2,4-Triazole der allgemeinen Formeln (VIII) und (IX)) oder vier Stickstoffatome (Tetrazole der allgemeinen Formeln (X) und (XI)) enthalten. Der in den allgemeinen Formeln (IV) bis (XI) jeweils an ein Ringstickstoffatom gebundene Rest $R^3$ steht für einen organischen, gegebenenfalls ein oder mehrere Heteroatome enthaltenden Rest,

für die $NH_2$-Gruppe und für ein Wasserstoffatom, vorzugsweise jedoch für ein Wasserstoffatom. Die in den allgemeinen Formeln (IV) bis (XI) an die Ringkohlenstoffatome gebundenen Reste $R^4$, $R^5$ und $R^6$ stehen für gleiche oder verschiedene, gegebenenfalls ein oder mehrere Heteroatome (z.B. N, O, S) aufweisende organische Reste, die gegebenenfalls Bestandteil eines weiteren, eventuell gemeinsamen Ringsystemes sein können, für die NH2-Gruppe und/oder für ein Wasserstoffatom. Als Kommponente C besonders bevorzugt sind solche Verbindungen der allgemeinen Formeln (IV) bis (XI), bei denen $R^3$ für ein Wasserstoffatom und $R^4$, $R^5$ und $R^6$ für gleiche oder verschiedene organische Reste mit 1-6 Kohlenstoffatomen und gegebenenfalls einem oder mehreren Heteroatomen, für die $NH_2$-Gruppe und/oder für ein Wasserstoffatom stehen. Beispielhaft, aber nicht ausschließlich geeignet, seien 1,2,4-Triazol. 3,5-Dimethyl-1,2,4-triazol, 3,5-Dimethylpyrazol und 1H-Imidazol genannt.

Die Herstellung der Mono- bzw. Polyasparaginsäureester der allgemeinen Formel (I) erfolgt durch Umsetzung der Komponente A mit Komponente B in Anwesenheit der Komponente C bei Temperaturen zwischen 0 und 100°C, vorzugsweise 20 - 80°C und besonders bevorzugt 20 - 60°C, wobei (i) das Verhältnis der Äquivalente der primären Aminogruppen der Komponente A zu den C=C-Doppelbindungsäquivalenten der Komponente B 1:2 bis 1,5:1, vorzugsweise jedoch 1:1,2 bis 1,2:1 beträgt,wobei überschüssige Edukte im Anschluß an die Reaktion abdestilliert werden können, und wobei (ii) der Anteil der erfindungsgemäß katalytisch wirksamen Komponente C am Reaktionsgemisch, bezogen auf den Festkörpergehalt des Reaktionsgemisches, 0,1 bis 10 Gew.-%, vorzugsweise 0,1 bis 5 Gew.-% und besonders bevorzugt 0,1 bis 2 Gew.-% beträgt. Die Reaktionsdauer kann in Abhängigkeit vom Mono- oder Polyamin und von den Anforderungen an den maximalen Restgehalt an Edukten im Reaktionsgemisch 8 h bis 4 Tage dauern.

Beispielhaft wird die Aminkomponente (Komponente A) zusammen mit 1 Gew.-% (bezogen auf den Festkörpergehalt des Gesamtansatzes aus Komponente A, Komponente B und Komponente C) der erfindungsgemäß katalytisch wirksamen Komponente C vorgelegt. Dieses Gemisch wird auf 60°C erwärmt und bei dieser Temperatur unter Rühren innerhalb von 8 h mit der Komponente B versetzt, wobei das Verhältnis der primären Aminogruppen der Komponente A zu den C=C-Doppelbindungen der Komponente B 1:1 beträgt. Nach Beendigung des Zutropfens läßt man noch 3 ½ - 4 Tage bei 60°C nachrühren und kühlt dann ab.

Als Bestandteil der einzelnen Komponenten A, B und C kommt sowohl jeweils eine einzelne Verbindung aus der jeweils betreffenden, oben genannten Verbindungsklasse als auch ein Gemisch aus zwei oder mehreren Verbindungen aus der jeweils betreffenden, oben genannten Verbindungsklasse in Frage.

Die erfindungsgemäße Herstellung der Mono- oder Polyasparaginsäureester der Formel (I) kann sowohl in Lösung als auch lösemittelfrei durchgeführt werden.

Der Zusatz von Lösemittel kann jedoch auch erst nach Beendigung des Syntheseprozesses erfolgen, z.B. um die Viskosität zu senken. Als Lösemittel kommen im Prinzip alle organischen Lösemittel in Frage, bevorzugt natürlich die in der Beschichtungstechnologie verwendeten Lösemittel. Beispielhaft, aber nicht ausschließlich gemeint, seien Aceton, Methylethylketon, Methylisobutylketon, n-Butylacetat, Methoxypropylacetat, Toluol, Xylol sowie höhere aromatische Lösemittel, wie sie von der Fa. Exxon-Chemie unter der Bezeichnung Solvesso® vertrieben werden, genannt.

Im Anschluß an die Synthese der Mono- und Polyasparaginsäureester lassen sich die Katalysatorkomponente C und damit das erfindungsgemäße Syntheseverfahren durch gaschromatographische Methoden noch nachweisen.

Die erfindungsgemäß erhaltenen Mono- und Polyasparaginsäureester können aufgrund des nahezu vollständigen Umsetzungsgrades direkt nach Beendigung des Syntheseverfahrens sofort als Reaktivkomponente für Polyisocyanate eingesetzt werden.

Eine erfindungsgemäße Verwendung der erfindungsgemäß erhaltenen Mono- und Polyasparaginsäureester besteht in einem Verfahren zur Herstellung von Überzügen durch Beschichtung beliebiger Substrate mit einem Beschichtungsmittel, welches als Bindemittel ein Zweikomponenten-System aus

a) einer Polyisocyanatkomponente und

b) einer gegenüber Polyisocyanaten reaktionsfähigen Reaktivkomponente

sowie gegebenenfalls die aus der Beschichtungstechnologie bekannten Hilfs- und Zusatzmittel enthält, wobei die Reaktivkomponente b) entweder aus

b1) einem oder mehreren, erfindungsgemäß erhaltenen Mono- oder Polyasparaginsäureestern der Formel (I)

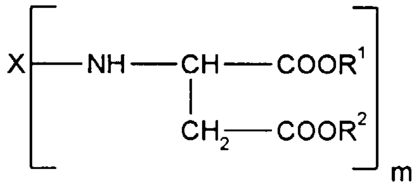

(I),

oder aus Gemischen derartiger Verbindungen mit

b2) aus der Polyurethanchemie an sich bekannten, gegenüber Isocyanatgruppen reaktionsfähigen Reaktivkomponenten besteht,

wobei X, $R^1$, $R^2$ und m die vorstehend genannten Bedeutungen haben.

Für das erfindungsgemäße Verfahren kommen als Polyisocyanatkomponenten a) im Prinzip alle aus der Polyurethanchemie bekannten Polyisocyanate in Betracht. Bevorzugt werden monomerenarme Derivate einfacher Polyisocyanate eingesetzt, wie sie in der Beschichtungstechnologie üblich sind. Bei diesen Derivaten handelt es sich um die an sich bekannten Biuretgruppen, Isocyanuratgruppen, Allophanatgruppen, Uretdiongruppen, Carbodiimidgruppen und/oder Urethangruppen aufweisenden Lackpolyisocyanate auf Basis von nachstehend genannten (cyclo)aliphatischen Polyisocyanaten. Als einfache Polyisocyanate gut geeignet sind niedermolekulare Polyisocyanate des Molekulargewichtsbereichs 140 bis 300 wie beispielsweise 1,4-Tetramethylendiisocyanat, 1,6-Hexamethylendiisocyanat (HDI), 2,2,4- und/oder 2,4,4-Trimethyl-hexamethylendiisocyanat, Dodecamethylendiisocyanat, 2-Methyl-1,5-diisocyanatopentan, 1,4-Diisocyanatocyclohexan, 1-Isocyanato-3,3,5-trimethyl-5-isocyanatomethylcyclohexan (IPDI), 2,4-und/oder 4,4'-Diisocyanatodicyclohexylmethan, 1-Isocyanato-1-methyl-3(4)-isocyanatomethylcyclohexan (IMCI), 2,4- und/oder 2,6-Hexahydrotoluylendiisocyanat ($H_6$TDI), 2,4- und/oder 4,4'-Diisocyanatodiphenylmethan oder Gemische dieser Isomeren mit ihren höheren Homologen, wie sie in an sich bekannter Weise durch Phosgenierung von Anilin/Formaldehyd-Kondensaten zugänglich sind, 2,4- und/oder 2,6-Diisocyanatotoluol und/oder beliebige Gemische derartiger Verbindungen. Selbstverständlich sind die aufgeführten, niedermolekularen Polyisocyanate, wenn auch weniger bevorzugt, auch als solche erfindungsgemäß verwendbar. Die entsprechenden Derivate von aromatischen Polyisocyanaten wie beispielsweise von 2,4- und/oder 2,6-Diisocyanatotoluol sind im Prinzip ebenfalls geeignet, jedoch weniger bevorzugt. Bei den genannten Urethangruppen aufweisenden Derivaten, d.h. den Urethangruppen aufweisenden Polyisocyanaten handelt es sich insbesondere um solche auf Basis von niedermolekularen Polyhydroxylverbindungen des Molekulargewichtsbereichs 62 bis 300 wie beispielsweise Ethylenglykol, Propylenglykol und/oder Trimethylolpropan.

Bei den besonders bevorzugt als Komponente a) geeigneten Lackpolyisocyanaten handelt es sich um Derivate der genannten Art auf Basis von 1,6-Hexamethylendiisocyanat mit einem NCO-Gehalt von 16 bis 24 Gew.-% und einer Viskosität bei 23°C von max. 10000, vorzugsweise max. 3000 mPas.

Bei der Komponente b1) handelt es sich um die erfindungsgemäß erhaltenen Mono- und Polyasparaginsäureester der Formel (I)

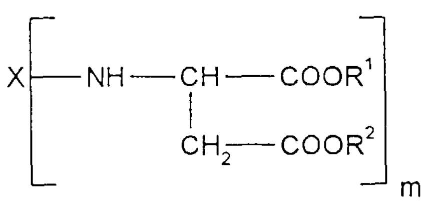

(I),

für welche X, $R^1$, $R^2$ und m die bereits vorstehend genannte Bedeutung haben.

Bevorzugt werden solche Verbindungen der Formel (I) verwendet, für welche m für 2 steht. Besonders bevorzugt sind jene, für welche X für einen zweiwertigen Kohlenwasserstoffrest steht, wie er durch Entfernung der Aminogruppen aus 1-Amino-3,3,5-trimethyl-5-aminomethylcyclohexan (IPDA), 4,4'-Diaminodicyclohexylmethan (PACM 20), 3,3'-Dimethyl-4,4'-diaminodicyclohexylmethan (Laromin C 260), Hexahydro-2,4- und/oder 2,6-diaminotoluol ($H_6$TDA), den isomeren C-Monomethyldiaminodicyclohexylmethanen oder aus 3(4)-Aminomethyl-1-methylcyclohexylamin (AMCA) erhalten wird.

Zu den bevorzugten Ausgangskomponenten b1) gehören im übrigen solche der genannten allgemeinen For-

mel (I), für welche $R^1$ und $R^2$, für eine Methyl-, Ethyl-, n- oder Isopropyl-, n-Butyl oder 2-Ethylhexylgruppe stehen.

Bei der gegebenenfalls zusätzlich verwendeten Komponente b2) handelt es sich um aus der Polyurethanchemie an sich bekannte, gegenüber Isocyanatgruppen reaktive Gruppen tragende Verbindungen, deren funktionelle Gruppen entweder direkt, unter Feuchtigkeitseinwirkung oder/und Wärmeeinwirkung mit Isocyanatgruppen abreagieren. Beispielhaft, aber nicht ausschließlich gemeint, seien genannt hydroxyfunktionelle Polyacrylate und Polyesterpolyole, wie sie in der EP-A 0 689 881 beschrieben sind, Oxazolidine, wie sie in der EP-A 0 639 628 beschrieben sind und Polyaldimine und Polyketimine, wie sie in der EP-A 0 699 696 beschrieben sind.

Im übrigen können als Komponente b2) beliebige Gemische der beispielhaft genannten, gegenüber Polyisocyanaten reaktionsfähigen Verbindungen verwendet werden.

In den erfindungsgemäß eingesetzten Bindemitteln werden im übrigen die Mengenverhältnisse der Komponenten a), b1) und (gegebenenfalls) b2) so bemessen, daß das Äquivalentverhältnis von Isocyanatgruppen zu gegenüber Isocyanatgruppen reaktionsfähigen bzw. potentiell reaktionsfähigen Gruppen bei 0,8:1 bis 20:1, vorzugsweise bei 0,8:1 bis 1,2:1 liegt. Bei der Berechnung der Äquivalentverhältnisse gehen die Oxazolidinringe der monocyclischen Mono- bzw. Polyoxazolidine als im Sinne der Isocyanat-Additionsreaktion potentiell difunktionelle Einheiten in die Berechnung ein.

Die Herstellung der erfindungsgemäß einzusetzenden Bindemittel geschieht durch Abmischung der Einzelkomponenten entweder lösemittelfrei oder in Anwesenheit der aus der Technologie der Polyurethanbeschichtungen üblichen Lösemittel.

Brauchbare Lösungsmittel sind beispielsweise Ethylacetat, Butylacetat, Methoxypropylacetat, Methylisobutylketon, Methylethylketon, Xylol, N-Methylpyrrolidon, Benzin, Chlorbenzole, ®Solvesso oder Gemische derartiger Lösemittel.

Im allgemeinen beträgt in den beim erfindungsgemäßen Verfahren einzusetzenden Beschichtungsmitteln das Gewichtsverhältnis der Gesamtmenge der Bindemittelkomponenten a) und b) zu Lösemittel 40:60 bis 100:0, vorzugsweise 60:40 bis 90:10.

In den beim erfindungsgemäßen Verfahren einzusetzenden Beschichtungsmitteln können auch andere, in der Beschichtungstechnologie übliche Hilfs- und Zusatzmittel enthalten sein. Hierzu gehören insbesondere Pigmente, Füllstoffe, Verlaufshilfsmittel, Katalysatoren, Antiabsetzmittel und usw..

Die Eigenschaften der beim erfindungsgemäßen Verfahren erhaltenen Überzüge können insbesondere durch geeignete Wahl von Art und Mengenverhältnissen der Ausgangskomponenten a), b1) und b2) eingestellt werden.

Zur Durchführung des erfindungemäßen Verfahrens werden die erfindungemäß zu verwendenden Beschichtungsmittel nach an sich bekannten Methoden, beispielsweise durch Spritzen, Streichen, Tauchen, Fluten oder mit Hilfe von Walzen oder Rakeln auf beliebige Substrate ein- oder mehrschichtig aufgetragen. Das erfindungsgemäße Verfahren eignet sich zur Herstellung von Überzügen auf beliebigen Substraten, wie z.B. Metallen, Kunststoffen, Holz oder Glas. Besonders gut geeignet ist das erfindungsgemäße Verfahren zur Herstellung von Überzügen auf Stahlblechen, wie sie beispielsweise bei der Herstellung von Fahrzeugkarosserien, Maschinen, Verkleidungsblechen, Fässern oder Containern Verwendung finden. Die beim erfindungsgemäßen Verfahren zu beschichtenden Substrate können vor der Durchführung des erfindungsgemäßen Verfahrens mit geeigneten Grundierungen versehen sein.

Die Trocknung der dem erfindungsgemäßen Verfahren entsprechenden Überzüge kann in einem weiten Temperaturintervall erfolgen, das von ca. 0 bis 160°C reicht.

Das oben eingehend beschriebene Verfahren zur Herstellung von Beschichtungen unter Verwendung der erfindungsgemäß hergestellten Mono- und Polyasparaginsäureester läßt sich ebenfalls auf die Darstellung von Harnstoff-, Urethan-, Allophanat- und/oder Biuretstrukturen aufweisenden Prepolymeren übertragen.

Bevorzugt werden hierbei als Komponente b1) solche erfindungsgemäß erhaltenen Mono- und/oder Polyasparaginsäureester der allgemeinen Formel (I), deren organischer Rest X eine oder mehrere weitere, bei Temperaturen bis 100°C gegenüber Isocyanatgruppen inerte funktionelle Gruppen beinhaltet. Diese funktionellen Gruppen bieten die Möglichkeit alternativer Vernetzungsmechanismen wie z.B. Feuchtigkeitshärtung. Beispielhaft, aber nicht ausschließlich, genannt seien erfindungsgemäß hergestellte, Alkoxy-, Alkyl- und/oder Alkoxyalkylsilangruppen aufweisende Monoasparaginsäureester der allgemeinen Formel (XII)

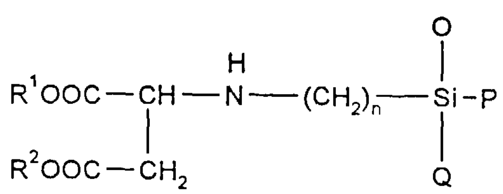

(XII),

in welcher

$R^1$ und $R^2$ die oben genannte Bedeutung haben,

n eine ganze Zahl ≥ 2 bedeutet und

O, P und Q gleiche oder verschiedene organische Reste bedeuten und für $C_1$-$C_4$-Alkyl- und/oder $C_1$-$C_{14}$-Alkoxyreste stehen können.

Die erfindungsgemäß erhaltenen Mono- und Polyasparaginsäureester sind direkt nach Abschluß des Syntheseprozesses einsatzfähig, da jetzt, im Gegensatz zu den ohne Katalyse dargestellten Mono- und Polyasparaginsäureestern, ein nahezu vollständiger Umsetzungsgrad erreicht ist. Durch den niedrigen Gehalt an Malein- bzw. Fumarsäureester und an primären Aminogruppen sind diese Produkte nicht nur toxikologisch und physiologisch unbedenklich, sondern zeigen auch gegenüber Isocyanatgruppen eine angemessene, nicht zu heftige Reaktivität. Aufgrund ihrer niedrigen Viskosität stellen sie als Reaktivverdünner eine mehr als geeignete Alternative für die bislang verwendeten, umweltbelastenden organischen Lösemittel dar und können somit als elementare Grundlage für hochwertige, lösemittelarme oder sogar -freie High Solid-2K-PUR-Lacke dienen.

Die folgenden Beispiele belegen die katalytische Wirkung von Fünfring-Heteroaromaten mit zwei oder mehreren Stickstoffatomen im Ringgerüst bei der Synthese von Mono- und Polyasparaginsäureestern durch Addition von primären Mono- und Polyaminen an Malein- oder Fumarsäureester.

Beschrieben werden die Umsetzungen mit und - zum Vergleich - ohne Zusatz der erfindungsgemäßen Katalysatoren, die jeweiligen Umsatzraten direkt nach Abschluß des Darstellungsverfahrens, die Eigenschaften der Polyasparaginsäureester (Viskosität, Farben) sowie ihr reaktives Verhalten gegenüber Polyisocyanaten (Gelierzeit, Filmbildung, Filmeigenschaften).

Alle Prozentangaben beziehen sich, sofern nicht anders lautend geschildert, auf das Gewicht.

## Beispiele

### A) Analytik der Polyasparaginsäureester

Die Bestimmung des Gehaltes an nicht umgesetztem Fumarsäureester (in den sich Maleinsäureester basenkatalytisch umlagert) erfolgte durch Supercritical Fluid Chromatography (SFC) mit dem Gerät SFC 3000 der Fa. Carlo Erba.

Bei den Werten handelt es sich um Angaben in Gewichtsprozent. Aus diesen Angaben läßt sich - bei Anwendung eines 1:1-Äquivalentverhältnisses primärer Aminogruppen zu C=C-Doppelbindungsäquivalenten des Malein- bzw. Fumarsäureesters - der Umsetzungsgrad nach folgender Formel berechnen:

$$\text{Umsetzungsgrad [\%]} = 100 - \frac{[FSE] \,.\, (n_A \,.\, Val_A + n_A \,.\, Val_{FSE} + M_c)}{n_A \,.\, Val_{FSE}}$$

$n_A$: Anzahl der Äquivalente an primären Aminogruppen bzw. Doppelbindungsäquivalente (Fumarsäureester)

$Val_A$: Äquivalentgewicht des Polyamins, bezogen auf $NH_2$-Gruppen, in g

[FSE]: Gehalt an nicht umgesetztem Fumarsäureester (FSE) in Gew.-%

$Val_{FSE}$: Äquivalentgewicht des Fumarsäureesters, bezogen auf C=C-Bindungen (⇒ 1 Val = 1 Mol) in g

$M_c$: eingesetzte Menge der Katalysatorkomponente C in g (= 0 g, wenn ohne Katalysator)

Da in den folgenden Beispielen ausschließlich primäre Diamine ($n_A$ = 2) und Fumarsäurediethylester (= FSDEE, $M_{FSDEE}$ = 172,18 g) eingesetzt werden, vereinfacht sich die vorstehend genannte Gleichung zu

$$\text{Umsetzungsgrad [\%]} = 100 - \frac{[FSDEE] \,.\, (2.\, Val_A + 344{,}36\text{ g} + M_c)}{344{,}36\text{ g}}$$

Die Viskositäten wurden mit einem Rotationsviskosimeter "Viskotester VT 181" der Fa. Haake bei 23 °C bestimmt.

Die erfindungsgemäßen Katalysatoren (in den genannten Beispielen: 1,2,4-Triazol, 3,5-Dimethyl-1,2,4-triazol und 3,5-Dimethylpyrazol) wurden nach gängigen Labormethoden dargestellt.

**B) Darstellung der Polyasparaginsäureester**

I) Allgemeine Vorschrift zur Herstellung der Polyasparaginsäureester ohne Katalyse (Vergleichsbeispiele)

In einem 1 l-Vierhalskolben mit Rührer, Tropftrichter, Rückflußkühler und Innenthermometer werden 2 Val eines Diamins (1 Mol) unter Stickstoffatmosphäre vorgelegt, auf 60°C erwärmt und bei dieser Temperatur innerhalb von 8 h unter Rühren mit 2 Val Maleinsäurediethylester (344,36 g; 2 Mol) versetzt. Anschließend läßt man 3 - 4 Tage bei 60°C nachrühren. Während der gesamten Reaktionszeit wird ein schwacher Stickstoffstrom über das Reaktionsgemisch geleitet.

II) Allgemeine Vorschrift zur erfindungsgemäßen Herstellung der Polyasparaginsäureester mit Katalyse durch 3,5-Dimethylpyrazol und 1,2,4-Triazole

Vorgehensweise wie unter I) beschrieben

Änderung: Katalysator wird zusammen mit dem Diamin vorgelegt.

Katalysatormenge: 1 Gew.-%, bezogen auf Festkörpergehalt des Reaktionsansatzes

**Beispiel 1 (Vergleichsbeispiel)**

| | |
|---|---|
| 238,4 g | 3,3'-Dimethyl-4,4'-diamino-dicyclohexylmethan (Laromin C 260®, BASF; 2 Val) |
| 344,4 g | Maleinsäurediethylester (MSDEE; 2 Val) |

| | |
|---|---|
| Reaktionstemperatur | 60°C |
| Reaktionsdauer | a) 1 Tag |
| | b) 2 Tage |
| | c) 4 Tage |

a) Nach einem Tag hat das Reaktionsgemisch eine Viskosität von 330 mPas/23°C. Der Gehalt an FSDEE beträgt 14 %, was einer Umsatzrate von 76,3 % entspricht. Nach 8 Monaten Lagerung bei Raumtemperatur ist ein Umsetzungsgrad von 94,4 % (= 3,3 % FSDEE) erreicht. Während dieser Zeit ist ein drastischer Viskositätsanstieg auf bis zu 2100 mPas/23°C sowie eine starke Gelbfärbung des vormals blaßgelben Produktes zu verzeichnen.

b) Nach 2 Tagen beträgt der FSDEE-Gehalt (in situ-Messung) 9,5 %, was einer Umsatzrate von 87,1 % entspricht.

c) Nach 4 Tagen wird ein FSDEE-Gehalt von 4,8 % gemessen, die Umsatzrate beträgt jetzt 93,5 %. Das so hergestellte Produkt hat eine Viskosität von 1110 mPas/23°C und ist stark gelb gefärbt.

In den nachstehend beschriebenen, erfindungsgemäßen Beispielen 2, 3 und 4 wird durch Zusatz von jeweils 1 Gew.-% an erfindungsgemäßem Katalysator eine höhere Umsatzrate direkt nach Beendigung des Syntheseprozesses erzielt. Die Viskositätswerte bilden einen weiteren Anhaltspunkt für die Vollständigkeit der Reaktion.

**Beispiel 2 (erfindungsgemäß, vgl. Bsp. 1)**

| | |
|---|---|
| 238,4 g | 3,3-Dimethyl-4,4'-diamino-dicyclohexylmethan (2 Val) |

(fortgesetzt)

| | |
|---|---|
| 344,4 g | Maleinsäurediethylester (2 Val) |
| 5,9 g | 1,2,4-Triazol als Katalysator (M = 69,07 g) |

| | |
|---|---|
| Reaktionszeit | 1 Tag |
| FSDEE-Gehalt | 7,6 % |
| Umsatzrate | 87,0 % |
| Viskosität: [mPas/23°C] | 750 |
| Farbe | blaßgelb |

**Beispiel 3 (erfindungsgemäß, vgl. Bsp. 1)**

| | |
|---|---|
| 238,4 g | 3,3-Dimethyl-4,4'-diamino-dicyclohexylmethan (2 Val) |
| 344,4 g | Maleinsäurediethylester (2 Val) |
| 5,9 g | 3,5-Dimethyl-1,2,4-triazol (M = 97,12 g) |

| | | | | | | |
|---|---|---|---|---|---|---|
| Reaktionszeit | a) | 1 Tag | b) | 2 Tage | c) | 4 Tage |
| FSDEE-Gehalt | | 9,2 % | | 6,0 % | | 3,3 % |
| Umsatzrate | | 84,3 | | 89,7 % | | 94,4 % |
| Viskosität [mPas/23°C] | | 630 | | - | | 1650 |
| Farbe | | farblos | | farblos | | blaßgelb |

**Beispiel 4 (erfindungsgemäß, vgl. Bsp. 1)**

| | |
|---|---|
| 238,4 g | 3,3-Dimethyl-4,4'-diamino-dicyclohexylmethan (2 Val) |
| 344,4 g | Maleinsäurediethylester (2 Val) |
| 5,9 g | 3,5-Dimethylpyrazol als Katalysator (M = 96,13 g) |

| | | | | | | |
|---|---|---|---|---|---|---|
| Reaktionszeit | a) | 1 Tag | b) | 2 Tage | c) | 4 Tage |
| FSDEE-Gehalt | | 8,1 % | | 5,8 % | | 3,8 % |
| Umsatzrate | | 86,2 % | | 90,1 % | | 93,5 % |
| Viskosität [mPas/23 °C] | | 630 | | - | | 1380 |
| Farbe | | farblos | | farblos | | blaßgelb |

Anhand der erfindungsgemäßen Beispiele 3 und 4 läßt sich erkennen, daß im Falle von Laromin C 260 als Diaminkomponente eine Reaktionszeit von 4 Tagen bei 60°C unter Verwendung der erfindungsgemäßen Katalysatoren ausreicht, um ein nahezu vollständig ausgereiftes Produkt zu erhalten. Dagegen liefert die herkömmliche Methode - 1 Tag bei 60°C, anschließend Lagerung bei Raumtemperatur - erst nach 4 - 8 Monaten Lagerungszeit ein Produkt mit entsprechenden Eigenschaften.

**Beispiel 5 (Vergleichsbeispiel zu den erfindungsgemäßen Beispielen 6 und 7)**

| | |
|---|---|
| 210, 4 g | 4,4'-Diaminodicyclohexylmethan (PACM 20; 2 Val) |
| 344,4 g | Maleinsäurediethylester (2 Val) |

| | | | | | | |
|---|---|---|---|---|---|---|
| Reaktionsdauer | a) | 1 Tag | b) | 2 Tage | c) | 4 Tage |
| FSDEE-Gehalt | | 7,26 % | | 4,7 % | | 2,67 % |
| Umsatzrate | | 88,2 % | | 92,3 % | | 95,7 % |
| Viskosität [mPas/23°C] | | 600 | | - | | 1350 |
| Farbe | | farblos | | blaßgelb | | blaßgelb |

a) Bricht man nach 1 Tag die Reaktion ab und lagert das Produkt anschließend bei Raumtemperatur, so wird erst nach 16 Wochen eine Umsatzrate von 97,9 % erzielt.

**Beispiel 6 (erfindungsgemäß, vgl. Bsp. 5)**

| | |
|---|---|
| 210, 4 g | 4,4'-Diaminodicyclohexylmethan (2 Val) |
| 344,4 g | Maleinsäurediethylester (2 Val) |
| 5,6 g | 3,5-Dimethyl-1,2,4-triazol als Katalysator |

| | | | | | | |
|---|---|---|---|---|---|---|
| Reaktionsdauer | a) | 1 Tag | b) | 2 Tage | c) | 4 Tage |
| FSDEE-Gehalt | | 5,6 % | | 3,5 % | | 2,8 % |
| Umsatzrate | | 90,9 % | | 94,3 % | | 95,4 % |
| Viskosität [mPas/23 °C] | | - | | - | | 1800 |
| Farbe | | farblos | | farblos | | blaßgelb |

**Beispiel 7 (erfindungsgemäß, vgl. Bsp. 5)**

| | |
|---|---|
| 210, 4 g | 4,4'-Diaminodicyclohexylmethan (2 Val) |
| 344,4 g | Maleinsäurediethylester (2 Val) |
| 5,6 g | 3,5-Dimethylpyrazol als Katalysator |

| | | | | | | |
|---|---|---|---|---|---|---|
| Reaktionsdauer | a) | 1 Tag | b) | 2 Tage | c) | 4 Tage |
| FSDEE-Gehalt | | 3,6 % | | 2,0 % | | 1,3 % |
| Umsatzrate | | 94,1 % | | 96,8 % | | 97,9 % |
| Viskosität [mPas/23°C] | | - | | - | | 1740 |
| Farbe | | farblos | | farblos | | blaßgelb |

**Beispiel 8 (Vergleichsbeispiel zu den erfindungsgemäßen Beispielen 9 und 10)**

| | |
|---|---|
| 128,2 g | 2,4- und 2,6-Hexahydrotoluylendiamin ($H_6$TDA, 80:20-Gemisch der 2,4- und 2,6-Isomeren; 2 Val) |
| 344,4 g | Maleinsäurediethylester (2 Val) |

| | | | | | | |
|---|---|---|---|---|---|---|
| Reaktionsdauer | a) | 1 Tag | b) | 2 Tage | c) | 4 Tage |
| FSDEE-Gehalt | | 13,9 % | | 9,6 % | | 6,3 % |
| Umsatzrate | | 80,7 % | | 86,7 % | | 91,3 % |
| Viskosität [mPas/23 °C] | | 180 | | - | | 195 |
| Farbe | | gelb | | tiefgelb | | tiefgelb |

**Beispiel 9 (erfindungsgemäß, vgl. Bsp. 8)**

| | |
|---|---|
| 128,2 g | 2,4- und 2,6-Hexahydrotoluylendiamin (2 Val) |
| 344,4 g | Maleinsäurediethylester (2 Val) |
| 4,8 g | 3,5-Dimethyl-1,2,4-triazol als Katalysator |

| | | | | | | |
|---|---|---|---|---|---|---|
| Reaktionsdauer | a) | 1 Tag | b) | 2 Tage | c) | 4 Tage |
| FSDEE-Gehalt | | 9,6 % | | 6,4 % | | 4,4 % |
| Umsatzrate | | 86,7 % | | 91,1 % | | 93,9 % |
| Viskosität [mPas/23 °C] | | - | | - | | 300 |
| Farbe | | gelb | | gelb | | tiefgelb |

**Beispiel 10 (erfindungsgemäß, vgl. Bsp. 8)**

| | |
|---|---|
| 128,2 g | 2,4- und 2,6-Hexahydrotoluylendiamin (2 Val) |
| 344,4 g | Maleinsäurediethylester (2 Val) |
| 4,8 g | 3,5-Dimethylpyrazol als Katalysator |

| | | | | | | |
|---|---|---|---|---|---|---|
| Reaktionsdauer | a) | 1 Tag | b) | 2 Tage | c) | 4 Tage |
| FSDEE-Gehalt | | 8,9 % | | 6,1 % | | 4,3 % |
| Umsatzrate | | 87,7 % | | 91,5 % | | 94,0 % |
| Viskosität [mPas/23 °C] | | - | | - | | 270 |
| Farbe | | gelb | | gelb | | tiefgelb |

**Beispiel 11**

**(Vergleichsbeispiel zu den erfindungsgemäßen Beispielen 2, 3 und 4)**

| | |
|---|---|
| 238,4 g | 3,3'-Dimethyl-4,4'-diamino-dicyclohexylmethan (2 Val) |
| 344,4 g | Maleinsäurediethylester (2 Val) |
| 5,9 g | 1,5-Diazabicyclo[4.3.0]non-5-en |

Nach einem Tag Reaktionszeit bei 60°C wurde in dem Reaktionsgemisch ein FSDEE-Gehalt von 7,9 % gefunden, was einer Umsetzungsrate von 86,5 % entspricht (Viskosität 540 mPas/23°C). Allerdings wies dieses Produkt im Gegensatz zu den erfindungsgemäßen Beispielen 2a, 3a und 4a (blaßgelb oder farblos) eine tiefgelborange Farbe auf.

C) **Lackkombinationen**

In den nachfolgend beschriebenen Beispielen werden Lackkombinationen aus den wie vorstehend beschrieben erhaltenen Polyasparaginsäureestern sowie einer Polyisocyanatkomponente beschrieben.

Zur Bestimmung der Gelierzeiten und zur Filmerzeugung wurden Abmischungen der einzelnen, erfindungsgemäß und vergleichsweise hergestellten Polyasparaginsäureester mit Desmodur N 3300® (handelsübliches, isocyanurathaltiges Polyisocyanat der Fa. Bayer AG; Isocyanatbasis: 1,6-Hexamethylendiisocyanat, Viskosität: 3000 mPas/23°C, NCO-Gehalt: 21,6 %, Äquivalentgewicht: 195 g) im Äquivalentverhältnis 1:1(NCO-Gruppen zu NCO-reaktiven Gruppen) hergestellt und mit Butylacetat auf einen Festkörpergehalt von 80 % gebracht.

Die Gelierzeit ist in den nachfolgend beschriebenen Beispielen als die Zeitspanne definiert, die nach Abmischung der Komponenten vergeht, bis das Gemisch nicht mehr rührbar ist.

Die Filme wurden mit einer Rakel auf vorher entfettete Glasplatten appliziert und bei Raumtemperatur getrocknet. Die Schichtdicke betrug in allen Fällen 90 µm; die Trocknung erfolgte bei Raumtemperatur.

Der Test auf die Lösemittelbeständigkeit eines Filmes besteht in einer einminütigen Einwirkung eines lösemittelgetränkten Wattebausches auf die Lackoberfläche (0 = Film unverändert, 5 = Film zerstört).

Der Test auf die Bleistifthärte eines Filmes besteht in Kratzversuchen mit Bleistiften unterschiedlicher Minenhärte. Angegeben wird die Härte, die mindestens erforderlich ist, um einen Kratzer auf der Filmoberfläche zu erzeugen (HB sehr weich, H = weich, 3H = sehr hart).

Äquivalentgewichte der Polyasparaginsäureester auf Basis von Maleinsäurediethylester und

(i) 3,3'-Dimethyl-4,4'-diaminodicyclohexylmethan (Bsp. 1 - 4, 11): ca. 292 g/NH
(ii) 4,4'-Diamindicyclohexylmethan (Bsp. 5 - 7): ca. 278 g/NH
(iii) 2,4- und 2,6-Hexahydrotoluylendiamin (Bsp. 8 - 10): ca. 237 g/NH

Bei den erfindungsgemäß dargestellten Polyasparaginsäureestern müssen diese Werte um den Faktor $\frac{1}{0{,}99}$ korrigiert werden (1 Gew.-% Katalysatorzusatz!).

<u>Tabelle 1</u>: Lackkombinationen aus Desmodur N 3300 und Polyasparaginsäureestern

| Beispiel-Nr. | 12 | 13 | 14 | 15 | 16 | 17 | 18 | 19 | 20 | 21 | 22 | 23 |
|---|---|---|---|---|---|---|---|---|---|---|---|---|
| Polyasparaginsäureester | | | | | | | | | | | | |
| aus Bsp.-Nr. | 1a | 1a* | 1c | 2 | 3c | 4c | 5c | 6c | 7c | 8c | 9c | 10c |
| Menge [g] | 292 | 292 | 292 | 295 | 295 | 295 | 278 | 281 | 281 | 237 | 240 | 240 |
| enthält Katalysator[1)] | - | - | - | 1,2,4-Triazol | DMT | DMP | - | DMT | DMP | - | DMT | DMP |
| Desmodur N 3300 [g] | 195 | 195 | 195 | 195 | 195 | 195 | 195 | 195 | 195 | 195 | 195 | 195 |
| Butylacetat [g] | 122 | 122 | 122 | 123 | 123 | 123 | 118 | 119 | 119 | 108 | 109 | 109 |
| Gelierzeit [h] | 21 | > 24 | < 24 | > 24 | > 24 | > 24 | 3 | 3,5 | 3,5 | > 8 | > 8 | > 8 |
| Lösemittelbeständigkeit | | | | | | | | | | | | |
| Methoxypropylacetat | 2 | 0 | 2 | 1 | 0 | 0 | 0 | 0 | 0 | 1 | 2 | 2 |
| Xylol | 2 | 0 | 0 | 0 | 0 | 0 | 0 | 0 | 0 | 1 | 1 | 1 |
| Essigsäureethylester | 2 | 0 | 1 | 0 | 0 | 0 | 0 | 0 | 0 | 1 | 3 | 2 |
| Aceton | 3 | 1 | 3 | 1 | 1 | 1 | 1 | 1 | 1 | 3 | 3 | 3 |
| Bleistifthärte | H | H | H | 2H | 2H | 2H | H | H | 2H | H | 2H | 2H |

* nach 8 Monaten Lagerung bei Raumtemperatur

1) DMT = 3,5-Dimethyl-1,2,4-triazol; DMP = 3,5-Dimethylpyrazol

## Patentansprüche

1. Verfahren zur Herstellung von sekundären Mono- und Polyaminen der allgemeinen Formel (I)

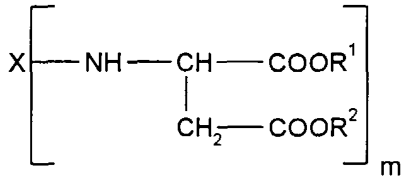
(I),

durch Umsetzung der entsprechenden Mono- oder Polyamine der allgemeinen Formel (II) (Komponente A)

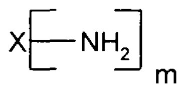
(II),

mit Verbindungen der allgemeinen Formel (III) (Komponente B)

$$R^1OOC\text{-}CH{=}CH\text{-}COOR^2 \quad (III),$$

bei Temperaturen zwischen 0°C und 100°C in Lösung oder lösemittelfrei, mit einem Verhältnis der Äquivalente der primären Aminogruppen der Komponente A zu den C=C-Doppelbindungsäquivalenten der Komponente B von 1:2 bis 1,5:1, wobei überschüssige Edukte im Anschluß an die Reaktion abdestilliert werden können, dadurch gekennzeichnet, daß die Reaktion in Anwesenheit einer katalytisch und verfärbungsstabilisierend wirkenden Komponente C, bestehend aus einer oder mehreren Verbindungen der allgemeinen Formeln (IV) bis (XI)

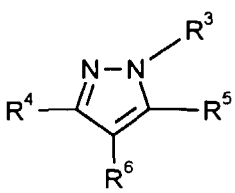
(IV),
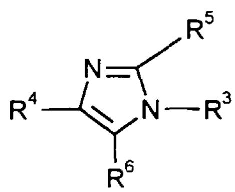
(V),
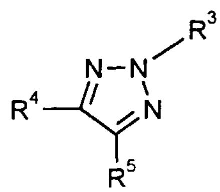
(VI),

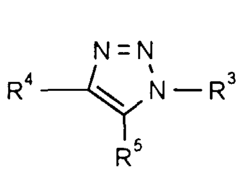
(VII),
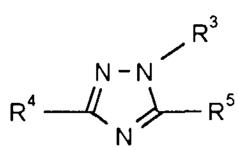
(VIII),
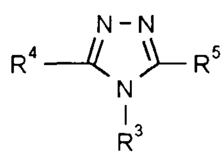
(IX),

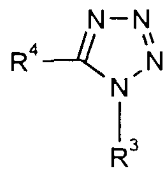

(X),

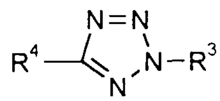

(XI),

abläuft,
wobei der Anteil der Komponente C am Reaktionsgemisch, bezogen auf den Festkörpergehalt des Reaktionsgemisches, 0,1 bis 10 Gew.-% beträgt, und

| | |
|---|---|
| X | für einen m-wertigen, gegebenenfalls ein oder mehrere Heteroatome enthaltenden organischen Rest steht, wie er durch Entfernung der primären Aminogruppe bzw. -gruppen aus einem entsprechenden, (cyclo)aliphatisch gebundene Aminogruppen aufweisenden Mono- oder Polyamin des Molekulargewichtsbereichs 60 bis 6000 erhalten werden kann, und der weitere gegenüber Isocyanatgruppen reaktive und/oder bei Temperaturen bis 100°C inerte funktionelle Gruppen enthalten kann, |
| $R^1$ und $R^2$ | für gleiche oder verschiedene organische Reste, |
| $R^3$ | für einen organischen, gegebenenfalls ein oder mehrere Heteroatome aufweisenden organischen Rest mit 1 bis 24 C-Atomen, für die $NH_2$-Gruppe oder für ein Wasserstoffatom steht, |
| $R^4$, $R^5$ und $R^6$ | für gleiche oder verschiedene, gegebenfalls ein oder mehrere Heteroatome aufweisende organische Reste mit 1 bis 24 C-Atomen, die gegebenenfalls Bestandteil eines weiteren, gegebenenfalls gemeinsamen Ringsystemes sein können, für die $NH_2$-Gruppe und/oder für ein Wasserstoffatom stehen, und |
| m | für eine ganze Zahl $\geq 1$ steht. |

**2.** Verfahren gemäß Anspruch 1, dadurch gekennzeichnet, daß man als Komponente B Verbindungen der allgemeinen Formel (III) verwendet, für welche $R^1$ und $R^2$ für eine Methyl-, Ethyl-, n- oder Isopropyl-, n-Butyl oder 2-Ethylhexylgruppe stehen.

**3.** Verfahren gemäß Anspruch 1, dadurch gekennzeichnet, daß man als Komponente A Verbindungen der allgemeinen Formel (II) verwendet, für welche X für den Rest steht, wie er durch Entfernung der Aminogruppe aus

a) Verbindungen des Typs $H_2N$- $R^7$-Si $R^8$ $R^9$ $R^{10}$, wobei $R^8$, $R^9$ und $R^{10}$ für ein Chloratom und/oder für gleiche oder verschiedene Alkyl- und/oder Alkoxyreste mit 1 bis 4 Kohlenstoffatomen und $R^7$ für einen Alkylrest mit 2 bis 6 Kohlenstoffatomen stehen,

b) Aminoalkoholen und

c) Ethylendiamin, 1,2-Diaminopropan, 1,4-Diaminobutan, 2,5-Diamino-2,5-dimethylhexan, 1,5-Diamino-2-methyl-pentan, 1,6-Diaminohexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethylcyclohexan, 2,4- und/oder 2,6-Hexahydrotoluylendiamin, anderen alkylsubstituierten Cyclohexandiaminen, wie z.B. Isopropyl-2,4- und/oder 2,6-diaminocyclohexan, 2,4'- und/oder 4,4'-Diamino-dicyclohexylmethan, 3,3'-Dimethyl-4,4'-diaminodicyclohexylmethan, den Isomeren, eine Methylgruppe als Kernsubstituenten aufweisenden Diaminodicyclohexylmethanen (= C-Monomethyl-diaminodicyclohexylmethane), 3- und/oder 4-Aminomethyl-1-methylcyclohexylamin und/oder höhermolekularen Polyetherpolyaminen mit aliphatisch gebundenen primären Aminogruppen, wie sie beispielsweise unter der Bezeichnung ®Jeffamin von der Firma Texaco vertrieben werden, erhalten werden kann.

**4.** Verfahren gemäß Anspruch 1, dadurch gekennzeichnet, daß man als Komponente C Verbindungen der allgemeinen Formel (IV) bis (XI) verwendet, für welche $R^3$ für ein Wasserstoffatom steht.

5. Verfahren gemäß Anspruch 1, dadurch gekennzeichnet, daß man als Komponente C Verbindungen der allgemeinen Formel (IV) bis (XI) verwendet, für welche $R^4$, $R^5$ und $R^6$ für gleiche oder verschiedene organische Reste mit 1 bis 6 Kohlenstoffatomen und gegebenenfalls einem oder mehreren Heteroatomen, für die $NH_2$-Gruppe und/oder für ein Wasserstoffatom stehen.

6. Verfahren gemäß Anspruch 1, dadurch gekennzeichnet, daß das Verhältnis der Äquivalente der primären Aminogruppen der Komponente A zu den C=C-Doppelbindungsäquivalenten der Komponente B 1:1,2 bis 1,2:1 beträgt.

7. Verfahren gemäß Anspruch 1, dadurch gekennzeichnet, daß der Anteil der Komponente C am Reaktionsgemisch, bezogen auf den Festkörpergehalt des Reaktionsgemisches, 0,1 - 2 Gew.-% beträgt.

8. Verfahren gemäß Anspruch 1, dadurch gekennzeichnet, daß die Umsetzung im Temperaturbereich von 20 - 80°C stattfindet.

9. Verwendung der gemäß Anspruch 1 erhaltenen Mono- und Polyamine der Formel (I) zur Herstellung von Überzügen durch Beschichtung beliebiger Substrate mit einem Beschichtungsmittel, welches als Bindemittel ein Zweikomponentensystem aus

a) einer Polyisocyanatkomponente und

b) einer gegenüber Polyisocyanaten reaktionsfähigen Reaktivkomponente

sowie gegebenenfalls die aus der Beschichtungstechnologie bekannten Hilfs- und Zusatzmittel enthält, wobei die Reaktivkomponente b) entweder aus

b1) einer oder mehreren, gemäß den Ansprüchen 1 bis 8 erfindungsgemäß erhaltenen Verbindungen der Formel (I)

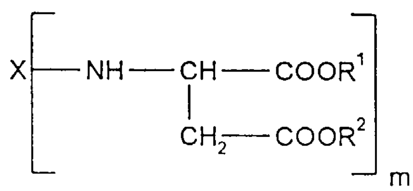

(I),

oder aus Gemischen derartiger Verbindungen mit

b2) aus der Polyurethanchemie an sich bekannten, gegenüber Isocyanatgruppen reaktionsfähigen Reaktivkomponenten besteht,

wobei

(i) die Komponenten b1) und b2) in beliebigem Mengenverhältnis zueinander stehen können,

(ii) das Äquivalentverhältnis von Isocyanatgruppen zu gegenüber Isocyanatgruppen reaktionsfähigen bzw. potentiell reaktionsfähigen Gruppen bei 0,8:1 bis 20:1 liegt und die Aushärtung nach erfolgter Applikation auf dem Substrat bei Temperaturen von - 20 bis + 100°C erfolgt und

(iii) in der allgemeinen Formel (I) X, $R^1$, $R^2$ und m die im Anspruch 1 genannten Bedeutungen haben.

10. Verwendung gemäß Anspruch 9, dadurch gekennzeichnet, daß X für den Rest steht, wie er durch Entfernung der Aminogruppen aus 1-Amino-3,3,5-trimethyl-5-aminomethylcyclohexan, 4,4'-Diaminodicyclohexylmethan, 3,3'-Dimethyl-4,4'-diaminodicyclohexylmethan, Hexahydro-2,4- und/oder 2,6-diaminotoluol, den isomeren C-Monomethyl-diaminodicyclohexylmethanen oder aus 3(4)-Aminomethyl-1-methylcyclohexylamin erhalten wird.

**11.** Verwendung gemäß Anspruch 9, dadurch gekennzeichnet, daß $R^1$ und $R^2$ die im Anspruch 2 genannten Bedeutungen haben.

**12.** Verwendung gemäß Anspruch 9, dadurch gekennzeichnet, daß m eine ganze Zahl ≥ 2 ist.

**13.** Verwendung gemäß Anspruch 9, dadurch gekennzeichnet, daß das Äquivalentverhältnis von Isocyanatgruppen der Komponente a) zu gegenüber Isocyanatgruppen reaktionsfähigen bzw. potentiell reaktionsfähigen Gruppen der Komponente b) bei 0,8:1 bis 1,2:1 liegt.

**14.** Verwendung der gemäß Anspruch 1 erfindungsgemäß erhaltenen Verbindungen der allgemeinen Formel (I) für sich allein oder im Gemisch mit einer oder mehreren, weiteren, gegenüber Isocyanatgruppen reaktionsfähigen Reaktivkomponenten als Reaktionspartner für Polyisocyanate bei der Herstellung von Harnstoff-, Urethan-, Allophanat- und/oder Biuretstrukturen aufweisenden Prepolymeren.

## Claims

**1.** A process for preparing secondary mono and polyamines of the formula (I),

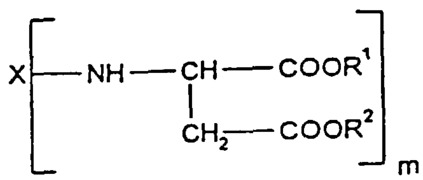

(I)

by reacting corresponding mono or polyamines of the general formula (II) (component A)

$$X[-NH_2]_m \qquad (II)$$

with compounds of the general formula (III) (component B)

$$R^1OOC\text{-}CH\text{=}CH\text{-}COOR^2 \qquad (III)$$

at temperatures between 0°C and 100°C, in solution or in the absence of a solvent, with a ratio by equivalents of primary amino groups in component A to C=C double bond equivalents in component B of 1:2 to 1.5:1, wherein excess reactants can be distilled out at the end of reaction,
characterised in that the reaction proceeds in the presence of a catalytically active and colour stabilising component C consisting of one or more compounds of the general formulas (IV) to (XI)

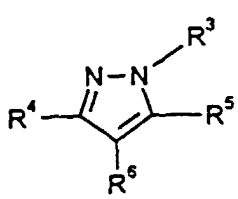

(IV),

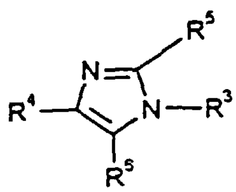

(V),

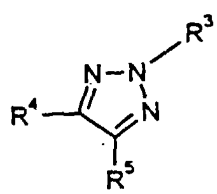

(VI),

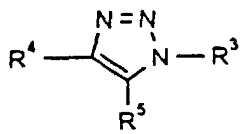

(VII),

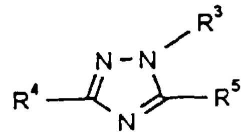

(VIII),

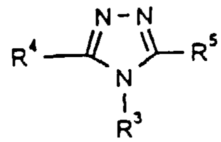

(IX),

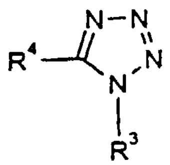

(X),

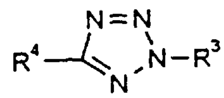

(XI),

wherein the proportion of component C in the reaction mixture, with reference to the solids content of the reaction mixture is 0.1 to 10 wt.%, and

| | |
|---|---|
| X | represents a m-valent organic group, optionally containing one or more heteroatoms, such as can be obtained by removing the primary amino group or groups from a corresponding mono or polyamine containing (cyclo)aliphatically bonded amino groups, with a molecular weight in the range 60 to 6000, and which may contain further functional groups which can react with isocyanate groups and/or are inert at temperatures up to 100°C, |
| $R^1$ and $R^2$ | represent identical or different organic groups, |
| $R^3$ | represents an organic group with 1 to 24 carbon atoms, optionally containing one or more heteroatoms, the $NH_2$ group or a hydrogen atom, |
| $R^4$, $R^5$ and $R^6$ | represent identical or different organic groups with 1 to 24 carbon atoms, optionally containing one or more heteroatoms, which may optionally be a constituent of a further, optionally common, ring system, the $NH_2$ group and/or a hydrogen atom, and |
| m | represents an integer ≥1. |

2. A process according to Claim 1, characterised in that compounds of the general formula (III) in which $R^1$ and $R^2$ represent a methyl, ethyl, n-propyl or isopropyl, n-butyl or 2-ethylhexyl group are used as component B).

3. Process according to Claim 1, characterised in that compounds of the general formula (II) in which X represents a group which can be obtained by removing the amino groups from

   a) compounds of the type $H_2N-R^7-Si-R^8R^9R^{10}$, wherein $R^8$, $R^9$ and $R^{10}$ represent a chlorine atom and/or identical or different alkyl and/or alkoxy groups with 1 to 4 carbon atoms and $R^7$ represents an alkyl group with 2 to 6 carbon atoms,

   b) aminoalcohols and

   c) ethylenediamine, 1,2-diaminopropane, 1,4-diaminobutane, 2,5-diamino-2,5-dimethylhexane, 1,5-diamino-2-methylpentane, 1,6-diaminohexane, 2,2,4- and/or 2,4,4-trimethyl-1,6-diaminohexane, 1,11-diaminoundecane, 1,12-diaminododecane, 1-amino-3,3,5-trimethyl-5-aminomethyl-cyclohexane, 2,4- and/or 2,6-hexahydrotoluylene diamine, other alkyl-substituted cyclohexane diamines such as, for example, isopropyl-2,4- and/or 2,6-diaminocyclohexane, 2,4'- and/or 4,4'-diaminodicyclohexyl-methane, 3,3'-dimethyl-4,4'-diaminocyclohexylmethane, the isomers of diaminodicyclohexylmethanes with a methyl group as ring-substituent (= C-monomethyldiaminodicyclohexylmethanes), 3- and/or 4-aminomethyl-1-methylcyclohexylamine and/or higher molecular weight polyetherpolyamines with aliphatically bonded primary amino groups such as are sold, for example, under the name ®Jeffamin by the Texaco Co.

are used as component A.

**4.** A process according to Claim 1, characterised in that compounds of the general formula (IV) to (XI) in which $R^3$ represents a hydrogen atom are used as component C.

**5.** A process according to Claim 1, characterised in that compounds of the general formula (IV) to (XI) in which $R^4$, $R^5$ and $R^6$ represent identical or different organic groups with 1 to 6 carbon atoms and optionally one or more heteroatoms, the $NH_2$ group and/or a hydrogen atom are used as component C.

**6.** A process according to Claim 1, characterised in that the ratio of equivalents of primary amino groups in component A to C=C double bond equivalents in component B is 1:1.2 to 1.2:1.

**7.** A process according to Claim 1, characterised in that the proportion of component C in the reaction mixture, with reference to the solids content of the reaction mixture, is 0.1 - 2 wt.%.

**8.** A process according to Claim 1, characterised in that the reaction is performed in the temperature range 20 - 80°C.

**9.** Use of the mono and polyamines obtained in accordance with Claim 1 to prepare surface coatings by coating any substrate with a coating agent which contains, as binder, a two-component system consisting of

a) a polyisocyanate component and

b) a reactive component capable of reacting with polyisocyanates

and optionally auxiliary substances and additives known from surface coating technology, wherein the reactive component b) consists of either

b1) one or more compounds of the formula (I) obtained in accordance with Claims 1 to 8

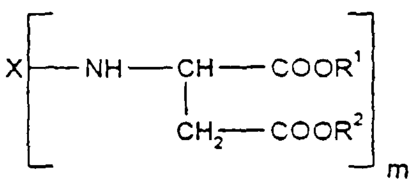

(I),

or a mixture of these types of compounds with

b2) reactive components which are capable of reacting with isocyanate groups known per se from polyurethane chemistry,

wherein

(i) components b1) and b2) may be present in any ratio by weight to each other,

(ii) the ratio by equivalents of isocyanate groups to groups capable of reacting with or potentially capable of reacting with isocyanate groups is 0.8:1 to 20:1 and curing is performed at temperatures from -20 to +100°C after application to the substrate and

(iii)X, $R^1$, $R^2$ and m in general formula (I) have the meanings given in Claim 1.

**10.** Use according to Claim 9, characterised in that X represents a group such as is obtained by removing the amino groups from 1-amino-3,3,5-trimethyl-5-aminomethylcyclohexane, 4,4'-diaminodicyclohexylmethane, 3,3'-dimethyl-4,4'-diaminodicyclohexylmethane, hexahydro-2,4- and/or 2,6-diaminotoluene, the isomers of C-monomethyldiaminodicyclohexylmethane or from 3(4)-aminomethyl-1-methylcyclohexylamine.

**11.** Use according to Claim 9, characterised in that $R^1$ and $R^2$ have the meanings given in Claim 2.

**12.** Use according to Claim 9, characterised in that m is an integer ≥2.

**13.** Use according to Claim 9, characterised in that the ratio by equivalents of isocyanate groups in component a) to groups capable of reacting with or potentially capable of reacting with isocyanate groups in component b) is 0.8:1 to 1.2:1.

**14.** Use of compounds of the general formula (I), obtained in accordance with Claim 1, on their own or mixed with one or more further reactive components capable of reacting with isocyanate groups, as reaction partners for polyisocyanates in the production of prepolymers with urea, urethane, allophanate and/or biuret structures.

## Revendications

**1.** Procédé pour la préparation de mono- et poly-amines secondaires de formule générale (I)

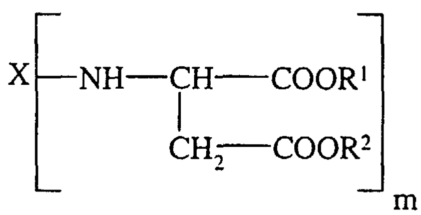

(I),

par réaction des mono- ou poly-amines correspondantes de formule générale (II) (composant A)

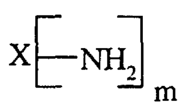

(II),

avec des composés de formule générale (III) (composant B)

$$R^1OOC\text{-}CH{=}CH\text{-}COOR^2 \quad \text{(III)},$$

à des températures de 0 à 100°C, en solution ou en l'absence de solvants, avec un rapport de 1:2 à 1,5:1 entre les équivalents de groupes amino primaires du composant A et les équivalents de doubles liaisons C=C du composant B, l'excès des produits de départ pouvant être éliminé par distillation après la réaction, caractérisé en ce que la réaction est réalisée en présence d'un composant C possédant une activité catalytique et stabilisatrice de couleur, qui consiste en un ou plusieurs composés de formules générales (IV) à (XI)

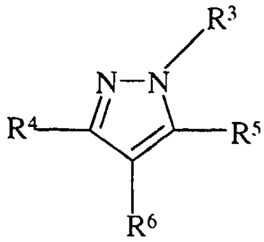

(IV),

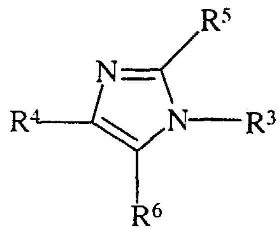

(V),

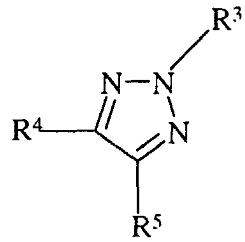

(VI),

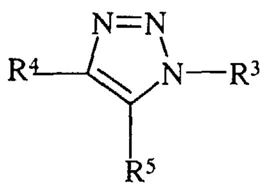

(VII),

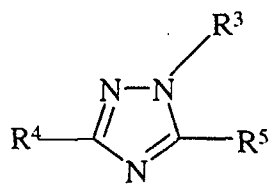

(VIII),

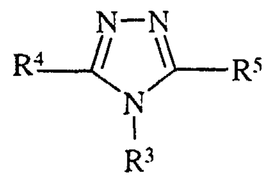

(IX),

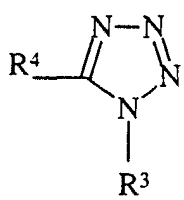

(X),

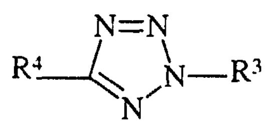

(XI),

la proportion du composant C dans le mélange de réaction, sur la teneur en matières solides totales du mélange de réaction, étant de 0,1 à 10 % en poids, et les symboles des formules ci-dessus ayant les significations suivantes :

| | |
|---|---|
| X | représente un radical organique de valence m, contenant le cas échéant un ou plusieurs hétéroatomes, tel qu'obtenu par élimination du ou des groupes amino primaires à partir d'une mono- ou poly-amine correspondante à groupes amino à liaisons (cyclo)aliphatiques de poids moléculaire 60 à 6000, et qui peut contenir d'autres groupes fonctionnels réactifs à l'égard des groupes isocyanate et/ou inertes à l'égard des groupes isocyanate à des températures allant jusqu'à 100°C, |
| $R^1$ et $R^2$ | représentent des radicaux organiques identiques ou différents, |
| $R^3$ | représente un radical organique en $C_1$-$C_{24}$ contenant le cas échéant un ou plusieurs hétéroatomes, un groupe $NH_2$ ou un atome d'hydrogène, |
| $R^4$, $R^5$ et $R^6$, | ayant des significations identiques ou différentes, représentent chacun un radical organique en $C_1$-$C_{24}$ contenant éventuellement un ou plusieurs hétéroatomes et qui peut le cas échéant être constituant d'un autre système cyclique éventuellement commun, un groupe $NH_2$ et/ou un atome d'hydrogène et |
| m | est un nombre entier ≥ 1. |

**2.** Procédé selon la revendication 1, caractérisé en ce que l'on utilise un composant B consistant en composés de formule générale (III) dans laquelle $R^1$ et $R^2$ représentent chacun un groupe méthyle, éthyle, n- ou isopropyle, n-butyle ou 2-éthylhexyle.

**3.** Procédé selon la revendication 1, caractérisé en ce que l'on utilise un composant A consistant en composés de

formule générale (II) dans laquelle X représente le radical obtenu par élimination du groupe amino de

a) des composés du type $H_2N$-$R^7$-Si $R^8R^9R^{10}$ dans laquelle $R^8$, $R^9$ et $R^{10}$ représentent des atomes de chlore ou des groupes alkyle et/ou alcoxy identiques ou différents en $C_1$-$C_4$ et $R^7$ représente un groupe alkyle en $C_2$-$C_6$,
b) d'aminoalcools et
c) de l'éthylènediamine, du 1,2-diaminopropane, du 1,4-diaminobutane, du 2,5-diamino-2,6-diméthylhexane, du 1,5-diamino-2-méthylpentane, du 1,6-diaminohexane, du 2,2,4- et/ou du 2,2,4-triméthyl-1,6-diaminohexane, du 1,11-diaminodécane, du 1,12-diaminododécane, du 1-amino-3,3,5-triméthyl-5-aminométhylcyclohexane, de la 2,4- et/ou de la 2,6-hexahydrotoluylènediamine, d'autres cyclohexanediamines à substituants alkyle, par exemple de l'isopropyl-2,4- et/ou 2,6-diaminocyclohexane, du 2,4'- et/ou du 4,4'-diaminodicyclohexylméthane, du 3,3'-diméthyl-4,4'-diaminodicyclohexylméthane, des diaminodicyclohexylméthanes isomères portant un groupe méthyle en tant que substituant cyclique (= C-monométhyl-diaminodicyclohexylméthanes), de la 3- et/ou de la 4-aminométhyl-1-méthylcyclohexylamine et/ou des polyétherpolyamines à haut poids moléculaire et à groupes amino primaires à liaisons aliphatiques, telles qu'elles existent par exemple dans le commerce sous la marque Jeffamin de la firme Texaco.

**4.** Procédé selon la revendication 1, caractérisé en ce que l'on utilise un composant C consistant en composés de formules générales (IV) à (XI) dans lesquelles $R^3$ représente un atome d'hydrogène.

**5.** Procédé selon la revendication 1, caractérisé en ce que l'on utilise un composant C consistant en composés de formules générales (IV) à (XI) dans lesquelles $R^4$, $R^5$ et $R^6$ représentent des radicaux organiques identiques ou différents en $C_1$-$C_6$ et qui contiennent le cas échéant un ou plusieurs hétéroatomes, un groupe $NH_2$ et/ou un atome d'hydrogène.

**6.** Procédé selon la revendication 1, caractérisé en ce que le rapport entre les équivalents de groupes amino primaires du composant A et les équivalents de doubles liaisons C=C du composant B va de 1:1,2 à 1,2:1.

**7.** Procédé selon la revendication 1, caractérisé en ce que la proportion du composant C dans le mélange de réaction, sur les matières solides totales du mélange de réaction, représente de 0,1 à 2 % en poids.

**8.** Procédé selon la revendication 1, caractérisé en ce que la réaction est réalisée à des températures de 20 à 80°C.

**9.** Utilisation des mono- et poly-amines de formule (I) obtenus selon la revendication 1, pour l'application de revêtements sur des supports quelconques à l'aide d'un produit de revêtement contenant en tant que liant un système à deux composants constitué lui-même de

a) un composant polyisocyanate et
b) un composant réactif à l'égard des polyisocyanates,

et le cas échéant les produits auxiliaires et additifs connus dans l'industrie des revêtements, le composant réactif b) consistant

b1) soit en un ou plusieurs composés de formule (I) obtenus conformément à l'invention selon les revendications 1 à 8,

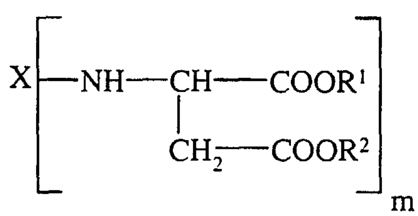

(I),

soit en mélanges de tels composés avec
b2) des composants réactifs avec les groupes isocyanate, connus en soi dans la chimie des polyuréthannes,

sous réserve que

(i) les composants b1) et b2) peuvent être présents dans des proportions relatives quelconques,
(ii) le rapport entre les équivalents de groupes isocyanate et les groupes réactifs ou potentiellement réactifs avec les groupes isocyanate va de 0,8:1 à 20:1 et le durcissement est réalisé après application sur le support à des températures allant de -20 à +100°C et
(iii) dans la formule générale (I), X, $R^1$, $R^2$ et m ont les significations indiquées dans la revendication 1.

**10.** Utilisation selon la revendication 9, caractérisée en ce que X représente un radical tel qu'obtenu par élimination des groupes amino du 1-amino-3,3,5-triméthyl-5-aminométhylcyclohexane, du 4,4'-diaminodicyclohexylméthane, du 3,3'-diméthyl-4,4'-diaminodicyclohexylméthane, de l'hexahydro-2,4- et/ou 2,6-diaminotoluène, des C-monométhyl-diaminodicyclohexylméthanes isomères ou de la 3(4)-aminométhyl-1-méthylcyclohexylamine.

**11.** Utilisation selon la revendication 9, caractérisée en ce que $R^1$ et $R^2$ ont les significations indiquées dans la revendication 2.

**12.** Utilisation selon la revendication 9, caractérisée en ce que m est un nombre entier $\geq 2$.

**13.** Utilisation selon la revendication 9, caractérisée en ce que le rapport entre les équivalents de groupes isocyanate du composant a) et les groupes réactifs ou potentiellement réactifs avec les groupes isocyanate du composant b) va de 0,8:1 à 1,2:1.

**14.** Utilisation des composés de formule générale (I) obtenus conformément à l'invention selon la revendication 1, isolément ou en mélange avec un ou plusieurs autres composants réactifs avec les groupes isocyanate, en tant que réactifs pour des polyisocyanates à la préparation de prépolymères à structures d'urée, d'uréthanne, d'allophanate et/ou de biuret.